# EUROPÄISCHE PATENTANMELDUNG

(11) **EP 1 488 823 A1**
(43) Veröffentlichungstag der Anmeldung: **22.12.2004**
(21) Anmeldenummer: 04090234.8
(22) Anmeldetag: 15.06.2004
(51) Int. Cl.: A61M 25/10, A61F 2/06

(54) **Ballonkatheter**

(30) Priorität: 21.06.2003 DE 10328817
(71) Anmelder: Biotronik GmbH & Co. KG, 12359 Berlin (DE)
(72) Erfinder: Wintsch, Christoph, 8311 Brütten (CH)
(74) Vertreter: Eisenführ, Speiser & Partner

(57) **Zusammenfassung**

Die Erfindung betrifft einen Ballonkatheter mit einem distalen Ende und einem proximalen Ende, umfassend einen äußeren Ballon am distalen Ende des Ballonkatheters mit einer äußeren Ballonwand und einer von der Ballonwand eingeschlossenen äußeren Ballonkammer, einen inneren Ballon mit einer inneren Ballonwand mit einer vorbestimmten Ballonwandstärke und einer Ballonwandoberfläche und einer von der inneren Ballonwand eingeschlossenen inneren Ballonkammer, dadurch gekennzeichnet, dass die Ballonkammern von der Katheterachse in radialer Richtung nach außen aufeinanderfolgend angeordnet sind und dass die innere Ballonwand eine Sollbruchstelle aufweist, welche ausgebildet ist, bei einem vorbestimmten Fluidüberdruck in der Ballonkammer des inneren Ballons zu zerreißen.

## Beschreibung

Die Erfindung betrifft einen Ballonkatheter mit einem distalen Ende und einem proximalen Ende. Der Ballonkatheter umfasst einen äußeren Ballon am distalen Ende des Ballonkatheters mit einer äußeren Ballonwand und einer von der Ballonwand eingeschlossenen äußeren Ballonkammer. Weiter umfasst der Ballonkatheter einen inneren Ballon mit einer inneren Ballonwand mit einer vorbestimmten Ballonwandstärke und einer Ballonwandoberfläche und einer von der inneren Ballonwand eingeschlossenen Ballonkammer. Aus dem Stand der Technik sind Ballonkatheter mit ein oder zwei Ballonen bekannt.

Aus der US 6,471,672 B1 ist ein Zwei-Ballonkatheter bekannt, bei welchem am distalen Ende auf einem Katheterrohr zwei Ballone angeordnet sind. Einer der zwei Ballone ist kleiner als der andere Ballon und für einen höheren Inflationsdruck ausgelegt. Der kleinere Ballon ist innerhalb einer von dem größeren Ballon eingeschlossenen Ballonkammer angeordnet. Der größere Ballon ist für einen kleineren Inflationsdruck ausgelegt und ist ausgebildet, einen Stent oder ein Medizinprodukt auf seiner äußeren Hülle zu tragen. Mit diesem Katheter wird ein Stent unter Inflation des äußeren Ballons vorexpandiert, wobei der innere Ballon ausgebildet ist, unter einem höheren Inflationsdruck den Stent in die endgültige Passform zu bringen.

Ein Anwendungsgebiet für Ballonkatheter ist die vaskuläre Intervention. Beispielsweise werden Ballonkatheter eingesetzt, um Stents in Koronargefäße einzusetzen. Die aus dem Stand der Technik bekannten Ballonkatheter weisen einen inflatierbaren Ballon auf, um einen in ein Gefäß eingebrachten Stent zu erweitern.

Ein Problem im Zusammenhang mit diesen Ballonkathetern ist, dass der Stent während des Inflationsvorgangs auf dem Ballon längsaxial verrutschen kann.

Die der Erfindung zugrunde liegende Aufgabe besteht darin, einen Ballonkatheter anzugeben, der ein einfaches und sicheres Positionieren insbesondere einer endoluminalen Prothese, beispielsweise in Form eines Stents erlaubt.

Diese Aufgabe wird erfindungsgemäß durch einen 2-Ballonkatheter der eingangs genannten Art gelöst, welcher sich dadurch auszeichnet, dass die Ballonkammern von der Katheterachse in radialer Richtung nach außen aufeinanderfolgend angeordnet sind und dass die innere Ballonwand eine Sollbruchstelle aufweist, welche ausgebildet ist, bei einem vorbestimmten Fluidüberdruck im Inneren des inneren Ballons zu zerreißen. Der Ballon ist dazu durch ein Fluid inflatierbar ausgebildet, wobei das Fluid beispielsweise eine physiologisch verträgliche Lösung sein kann.

An der gewünschten Position im Gefäß kann der Stent vorteilhaft durch Inflation des inneren Ballons vorerst auf dem Katheter fixiert werden. Der innere Ballon wird im Folgenden Fixierungsballon genannt. Der Fixierungsballon ist ausgebildet, den Stent auf einem oder mehreren Teilabschnitten des Stents in axialer Richtung partiell zu erweitern. Der eigentliche Öffnungsvorgang des Stents kann durch den Öffnungsballon erfolgen. Die innere Ballonwand umfasst eine Sollbruchstelle, welche ausgebildet ist, bei einem vorbestimmten Fluidüberdruck im Inneren des inneren Ballons zu zerreißen. Das Katheterrohr kann dadurch vorteilhaft nur ein Lumen umfassen, über welches sowohl der Fixierungsballon als auch der Öffnungsballon inflatiert wird.

In einer bevorzugten Ausführungsform des Ballonkatheters ist der innere Ballon in seiner zur Katheterlängsachse parallelen Achsrichtung kürzer als der äußere Ballon. Weiter vorteilhaft ist der innere Ballon auf der Katheterlängsachse im Zentrum des äußeren Ballons angeordnet. Wird der innere Ballon, im Vorgenannten auch als Fixierungsballon bezeichnet, zuerst inflatiert, wird ein auf dem äußeren Ballon aufgebrachter Stent nur in einem Teilabschnitt in längsaxialer Richtung im Bereich des Fixierungsballons erweitert. Der durch den Fixierungsballon teilweise erweiterte Stent ist in dieser Ausführungsform des Ballonkatheters durch einen Kraft-Formschluss gegen längsaxiale Verschiebung gesichert. Der äußere Ballon, im Folgenden Öffnungsballon genannt, kann nun inflatiert werden, um den darauf aufgebrachten Stent auf den gewünschten Durchmesser zu erweitern. Während dieses Öffnungsvorgangs ist der Stent in längsaxialer Richtung vorteilhafterweise gegen unbeabsichtigtes Verrutschen gesichert.

In einer weiter bevorzugten Ausführungsform des Ballonkatheters befindet sich die innere Ballonwand vollständig in der äußeren Ballonkammer. In dieser Ausführungsform ist der Fixierungsballon in dem Öffnungsballon eingeschlossen. In dieser Ausführungsform wird der Öffnungsballon durch den Fixierungsballon nicht beeinträchtigt. Das Katheterrohr kann in dieser Ausführungsform zwei jeweils voneinander getrennte röhrenförmige Lumina umfassen, welche jeweils fluiddynamisch mit jeweils einer der Ballonkammern verbunden sind. Über diese Lumina können der Öffnungsballon und der Fixierungsballon jeweils unabhängig voneinander inflatiert werden.

Weiter bevorzugt umfasst der Ballonkatheter eine Sollbruchstelle mit einem punkt- und/oder linienförmigen Bereich der Ballonwandoberfläche der inneren Ballonwand, wobei der punkt- und/oder linienförmige Bereich der Ballonwandoberfläche eine kleinere Ballonwandstärke aufweist als der die Sollbruchstelle umgebende Bereich der Ballonwandoberfläche. Durch derartige Ausbildung einer Sollbruchstelle wird erreicht, dass der Ballon bevorzugt an dieser Sollbruchstelle reißt. Weiter bevorzugt umfasst der Ballonkatheter eine Sollbruchstelle, welche bei einem Fluidüberdruck im Inneren des Ballons von 4-6 Bar zerreißt. Besonders bevorzugt zerreißt die Sollbruchstelle bei einem Fluidüberdruck im Inneren des Ballons von 4,5 - 5,5 Bar. Der Fluidüberdruck bezieht sich auf den umgebenden Luftdruck. Die Ballone können auch ausgebildet sein, bei Füllung mit einem Fluid die Ballonwandoberfläche zu vergrößern und die Ballonwandstärke zu verkleinern.

In einer alternativen Ausführungsform umfasst der Fixierungsballon mindestens ein Ventil, welches ausgebildet ist, bei einem vorbestimmten Fluidüberdruck im Inneren des inneren Ballons die innere Ballonkammer nach außen zu öffnen. Durch ein solches Ventil wird vorteilhaft erreicht, dass der Überdruck im Fixierungsballon allmählich entweicht. Ein solches Ventil kann zum Einsatz kommen, wenn der äußere Ballon oder das medizinische Gerät, welches auf dem äußeren Ballon befestigt ist, durch einen plötzlichen Druckanstieg gefährdet werden könnte. Das Ventil umfasst vorzugsweise eine Öffnung und einen die Öffnung verschließenden Stift, wobei das Ventil ausgebildet ist, den Stift bei einem vorbestimmten Fluiddruck im Inneren des Fixierungsballons aus der Öffnung nach außen freizugeben. Bei dieser Variante des Ventils kann der Stift in der Öffnung kraftschlüssig, oder kraftschlüssig und formschlüssig eingebunden sein. Die Öffnung kann dazu beispielsweise ausgebildet sein, bis zu einem bestimmten Druck im Inneren des Ballons den die Öffnung verschließenden Stift formschlüssig zu halten. Oberhalb eines vorbestimmten Fluiddruckes im Inneren des Ballons ist die Öffnung ausgebildet, sich derart auszudehnen, dass der Formschluss gelöst wird und der die Öffnung verschließende Stift freigegeben wird. Der Fluidüberdruck im Inneren des Ballons kann entweichen. In allen zuvor beschriebenen Ausführungsformen können die Ballone jeweils aus verschiedenen Materialien zusammengesetzt sein. Geeignete Ballonmaterialien können die Stoffe Polyolefincopolymer, Polyesther, Polyethylen, Polyether-Amid, Polyamid, Nylon oder andere Urethane beinhalten. Besonders bevorzugt beinhaltet das Ballonmaterial Latex. In allen zuvor beschriebenen Ausführungsformen kann der Katheter eine verschiebliche Hülle umfassen, welche über den auf dem Öffnungsballon angebrachten Stent geschoben ist und ausgebildet ist, den Stent zurückziehbar freizugeben. Die Hülle kann Silikon enthalten.

Die Erfindung soll nun anhand von Figuren näher erläutert werden.
- Fig. 1: zeigt ein distales Ende eines Ballonkatheters mit zwei Ballonen und einem Stent;
- Fig. 2: zeigt das distale Ende eines Zwei-Ballonkatheters mit einem aufgebrachten Stent und inflatiertem Fixierungsballon
- Fig. 3 und 4: zeigen zwei Ausführungsformen eines Ventils in einer Ballonwand

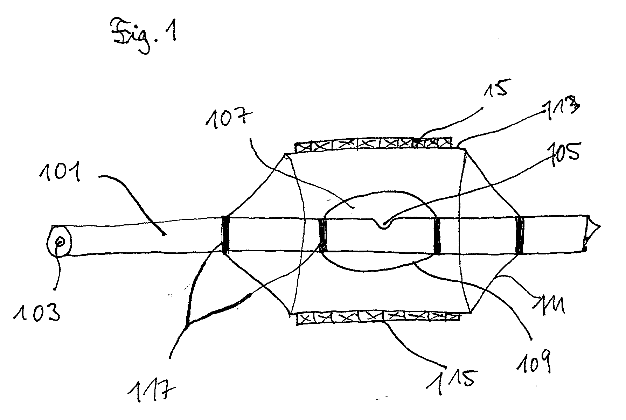

Figur 1 zeigt das distale Ende eines Zwei-Ballon katheters. Das Katheterende umfasst ein Katheterrohr 101, welches ein Lumen 103 aufweist, das fluiddynamisch über eine Rohröffnung 105 mit einer Ballonkammer 107 eines inneren Fixierungsballons 109 in Verbindung steht. Das Katheterende umfasst weiter einen Öffnungsballon 111, welcher eine Aufnahmefläche 113 zur Aufnahme eines Medizinproduktes, bevorzugt eines Stents 115 aufweist. Die Ballonenden sind an Dichtstellen 117 fluiddicht und ausreichend zugbelastungsfest an dem Katheterrohr 101 befestigt. Die Ballonwand kann an der Dichtstelle 117 durch Schweißung, Klebung, oder durch Kraft- und Formschluss am Katheterrohr 101 angebracht sein. Eine kraft- und formschlüssige Verbindung kann beispielsweise an dem Katheterrohr eine Nut umfassen, in welche das Ballonwandmaterial eingepresst ist. Zur weiteren Fixierung können zusätzliche Haltemittel von oben auf die Ballonwand in die Nut eingepresst sein. Ein solches Haltemittel kann beispielsweise ein O-Ring sein.

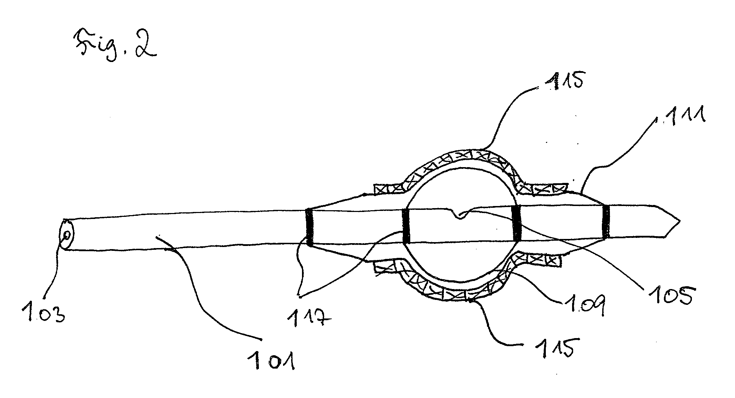

Figur 2 zeigt das in Figur 1 dargestellte distale Ende eines Zwei-Ballonkatheters mit inflatiertem. Ein Stent 115 ist durch den inflatierten Fixierungsballon 109 kraft- und formschlüssig gegen axiale Verschiebung auf dem Öffnungsballon 111 gesichert.

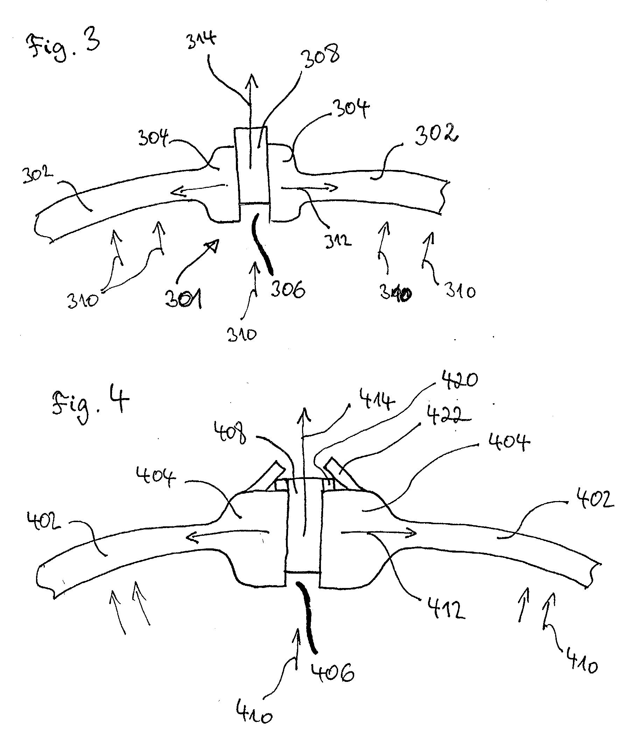

Figur 3 zeigt einen Querschnitt eines Ausschnitts eines Fixierungsballones 109. Das Ventil 301 ist in die Ballonwand 302 integriert, oder eingebracht. Das Ventil 301 umfasst einen Ventilsitz 304 und eine Ventilöffnung 306, in welche ein Verschlussstopfen 308 kraftschlüssig eingebracht ist. Der Ventilsitz 304 ist ausgebildet, durch die an diesen angebundene Ballonwand 302 in Richtung 312 mitgedehnt zu werden. Wenn der Fluidüberdruck, in der Abbildung dargestellt durch die Pfeile 310, einen vorbestimmten Wert erreicht, vergrößert sich die Öffnung 306, so dass der Verschlussstopfen 308 durch den Fluiddruck 310 in Richtung außen 314 befördert wird. Der Ventilsitz 304 und die Ballonwand 302 sind dazu elastisch ausgebildet.

Figur 4 zeigt ein Ventil analog zu Figur 3. Die Bezugszeichen entsprechen denen der Figur 3, jeweils um Einhundert erhöht. Das in Figur 4 gezeigte Ventil weist zusätzlich zu dem Ventil in Figur 3 einen formschlüssigen Verschluss des Ventilstopfens 408 mit dem Ventilsitz 404 auf. Das Ventil umfasst dazu einen elastisch ausgebildeten Niederhalter 422. Der elastische Niederhalter 422 kann auch um die Öffnung 406 umlaufend als Niederhaltungsring ausgebildet sein. Zum Bewirken eines Formschlusses mit dem Niederhalter 422 kann der Verschlussstopfen 408 auch einen Überstand 420 aufweisen. Der Überstand 420 kann auch als umlaufender Überstandsring ausgebildet sein. Alternativ dazu kann der Niederhalter 420 auch direkt auf die Oberkante des in Figur 3 gezeigten Stopfens 308 aufliegen, und somit einen Formschluss erzeugen. Wird der zwischen dem Ventilsitz 404 und dem Ventilstopfen 408 zustande kommende Reibschluss durch die Dehnung 412 verringert, kann der Verschlussstopfen 408 bei einem bestimmten Innendruck 410 sich in die äußere Richtung 414 bewegen. Zur Einstellung des Öffnungsdruckes sind die Niederhalter 422 mit entsprechender Elastizität versehen. Der in Figur 3 und Figur 4 gezeigte Verschlussstopfen 308 bzw. 408 kann auch kugelförmig oder mit einem ellipsenförmigen Schnitt oder rugbyballförmig ausgebildet sein.

## Patentansprüche

1. Ballonkatheter mit einem distalen Ende und einem proximalen Ende, umfassend:
einen äußeren Ballon am distalen Ende des Ballonkatheters mit einer äußeren Ballonwand und einer von der Ballonwand eingeschlossenen äußeren Ballonkammer,
einen inneren Ballon mit einer inneren Ballonwand mit einer vorbestimmten Ballonwandstärke und einer Ballonwandoberfläche und einer von der inneren Ballonwand eingeschlossenen inneren Ballonkammer,
**dadurch gekennzeichnet, dass**
die Ballonkammern von der Katheterachse in radialer Richtung nach außen aufeinanderfolgend angeordnet sind und dass die innere Ballonwand eine Sollbruchstelle aufweist, welche ausgebildet ist, bei einem vorbestimmten Fluidüberdruck in der Ballonkammer des inneren Ballons zu zerreissen.

2. Ballonkatheter nach Anspruch 1, **dadurch gekennzeichnet, dass** der innere Ballon in seiner zur Katheterlängsachse parallelen Achsrichtung kürzer ist als der äußere Ballon.

3. Ballonkatheter nach Anspruch 1 oder 2, **dadurch gekennzeichnet, dass** der innere Ballon auf der Katheterlängsachse im Zentrum des äußeren Ballons angeordnet ist.

4. Ballonkatheter nach Anspruch 2 oder 3, **dadurch gekennzeichnet, dass** die innere Ballonwand sich vollständig in der äußeren Ballonkammer befindet.

5. Ballonkatheter nach einem der vorhergehenden Ansprüche, **dadurch gekennzeichnet, dass** die Sollbruchstelle einen punkt- und/oder linienförmigen Bereich der Ballonwandoberfläche der inneren Ballonwand umfasst, wobei der punkt- und/oder linienförmige Bereich der Ballonwandoberfläche eine kleinere Ballonwandstärke aufweist als der die Sollbruchstelle umgebende Bereich der Ballonwandoberfläche.

6. Ballonkatheter nach einem der vorhergehenden Ansprüche, **dadurch gekennzeichnet, dass** die Sollbruchstelle bei einem Fluidüberdruck im Inneren des Ballons von 4 bis 6 Bar zerreißt.

7. Ballonkatheter nach einem der vorhergehenden Ansprüche, **dadurch gekennzeichnet, dass** die Sollbruchstelle bei einem Fluidüberdruck im Inneren des Ballons von 4,5 bis 5,5 Bar zerreißt.

8. Ballonkatheter nach einem der vorhergehenden Ansprüche, **dadurch gekennzeichnet, dass** das Ballonwandmaterial Latex enthält.

9. Ballonkatheter mit einem distalen Ende und einem proximalen Ende, umfassend:
einen äußeren Ballon am distalen Ende des Ballonkatheters mit einer äußeren Ballonwand und einer von der Ballonwand eingeschlossenen äußeren Ballonkammer,
einen inneren Ballon mit einer inneren Ballonwand mit einer vorbestimmten Ballonwandstärke und einer Ballonwandoberfläche und einer von der inneren Ballonwand eingeschlossenen Ballonkammer,
**dadurch gekennzeichnet, dass**
die Ballonkammern von der Katheterachse in radialer Richtung nach außen aufeinanderfolgend angeordnet sind und dass der innere Ballon mindestens ein Ventil umfasst, welches ausgebildet ist, bei einem vorbestimmten Fluidüberdruck in der Ballonkammer des inneren Ballons nach außen zu öffnen.

10. Ballonkatheter nach Anspruch 10, **dadurch gekennzeichnet, dass** das Ventil eine Öffnung und einen die Öffnung verschließenden Stift umfasst, und das Ventil ausgebildet ist, den Stift bei einem vorbestimmten Fluidüberdruck in der Ballonkammer des inneren Ballons aus der Öffnung freizugeben.
